# EUROPEAN PATENT APPLICATION

(11) **EP 1 582 159 A1**
(43) Date of publication of application: **05.10.2005**
(21) Application number: 04007785.1
(22) Date of filing: 31.03.2004
(51) Int. Cl.: A61B 17/72

(54) **Intramedullary nail provided with expansion means for fixation to the bone**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera del Garda (Verona) (IT); Venturini, Daniele, 37064 Povegliano Veronese (Verona) (IT)
(74) Representative: Ferreccio, Rinaldo

(57) **Abstract**

An intramedullary nail (10) suitable for insertion in a fractured elongate bone (12) and for an unusually simple fixation therein comprises a substantially straight stem (14, 114, 214, 314) extending between a proximal end (16, 116, 216, 316) and a distal end (18, 118, 218, 318), one of said ends (16, 18; 116, 118; 216, 218; 316, 318) comprising expansion means (20, 120, 220, 320) for the nail fixation to the bone, said expansion elements (20, 120, 220, 320) comprising at least an element (20a, 120a, 220a, 320a) realised with a shape-memory material.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem extending between a proximal end and a distal end, at least one of said ends comprising expansion means for the nail fixation to the bone.

### Prior art

Intramedullary nails are known, which, during a surgical operation, are inserted in a fractured elongate bone and fixed therein, in order to reconstruct the original bone shape and in the meantime to recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two similar offset holes, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly occurring when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

An intramedullary nail being fixed without using bone screws is also known from the international patent application no. WO 0134045,

The fixation is performed through a radial expansion of two nail portions, positioned near the distal and proximal end of the nail stem. In other words, at least one of said ends comprises expansion means for the nail fixation to the bone.

These portions are sleeves provided with longitudinal slots: the sleeve radial expansion is performed by applying an axial compression mechanical action to the ends of said sleeves. The compression is performed through an axial movement of a further stiff sleeve, which is moved by screwing it by hand on the stem.

Although advantageous under different points of view with respect to the above-mentioned prior art, the intramedullary nail structured as schematically above described has well-known drawbacks.

First, rotations of the nail in the bone can actually occur, since only the friction of the expansion portions against the bone causes the nail fixation.

Moreover, as a result of the expansion step of the two nail portions, with the subsequent shortening thereof in the axial direction, an undesired shortening of the bone whereon operation occurs is obtained, since the two sleeves equipped with slots are approaching to each other.

Still further, in the case of bone lyses in the contact area between the expanded sleeves and the bone, the nail fixation becomes insecure and it is necessary to operate from the outside to recover, as much as possible, a proper pressure in this contact area.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that said expansion means comprise at least an element realised with a shape-memory material.

Further features and the advantages of the intramedullary nail suitable for insertion in a fractured elongate bone, as well as of the application method of said nail in said bone, according to the present invention, will be apparent from the following description of an embodiment thereof made with reference to the attached drawings, given by way of nonlimiting example.

### Brief description of the drawings

Figure 1 is a schematic perspective view of a first embodiment of an intramedullary nail, suitable for insertion in a fractured elongate bone, according to the present invention.
Figure 2a is a schematic perspective view of the nail of figure 1.
Figure 2b is a schematic perspective view of the nail of figure 2a, in a different shape.
Figure 3a is a further schematic enlarged perspective view of the nail of figure 1.
Figure 3b is a schematic perspective view of the nail of figure 3a, in a different shape.
Figure 4 is a schematic perspective view of a portion of the nail of figure 1.
Figure 5 is a schematic perspective view of an alternative embodiment of an intramedullary nail according to the present invention.
Figure 5a is a schematic plan view of an alternative embodiment of the nail of figure 5.
Figure 5b schematically shows a side-elevation cross-section of the nail of figure 5a, taken according to the traced V-V plane.
Figure 6 is a schematic perspective view of a further alternative embodiment of the nail of figure 5.
Figure 7a is a schematic perspective view of an alternative embodiment of an intramedullary nail according to the present invention.
Figure 7b is a schematic perspective view of the nail of figure 7a, in a different shape.
Figure 8 is a schematic perspective view of an alternative embodiment of an intramedullary nail according to the present invention.
Figure 9 is a schematic enlarged perspective view of the nail of figure 8.
Figure 10 is a further schematic enlarged perspective view of the nail of figure 8.
Figure 11 is a schematic perspective view of a portion of the nail of figure 8.

### Detailed description

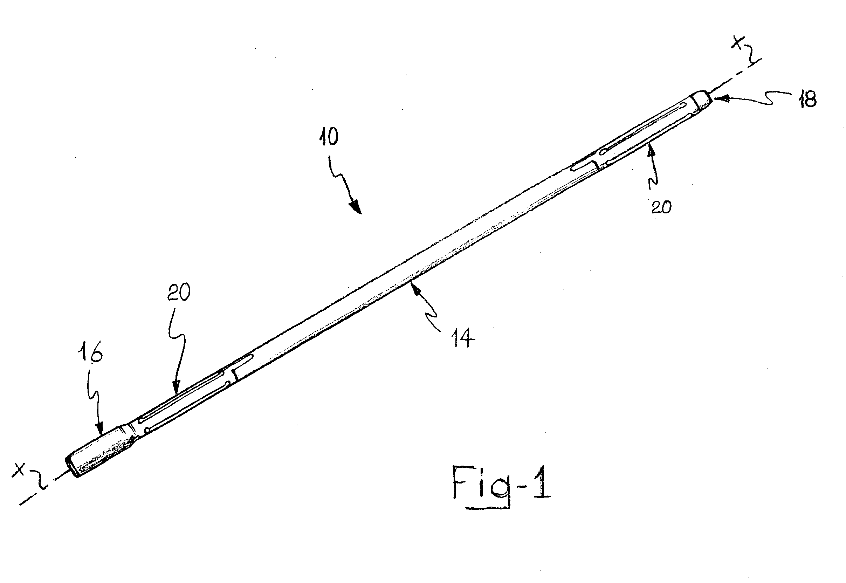

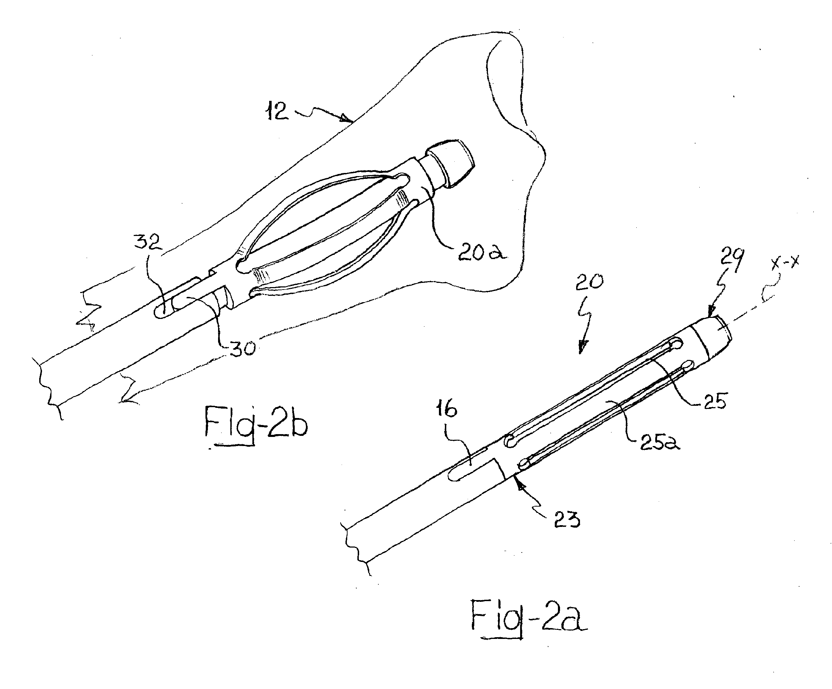

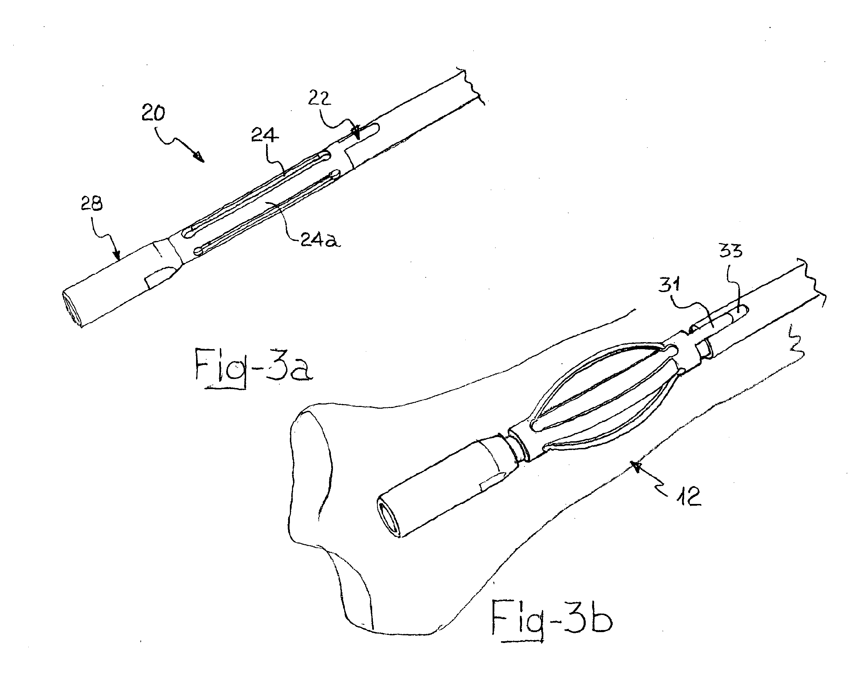

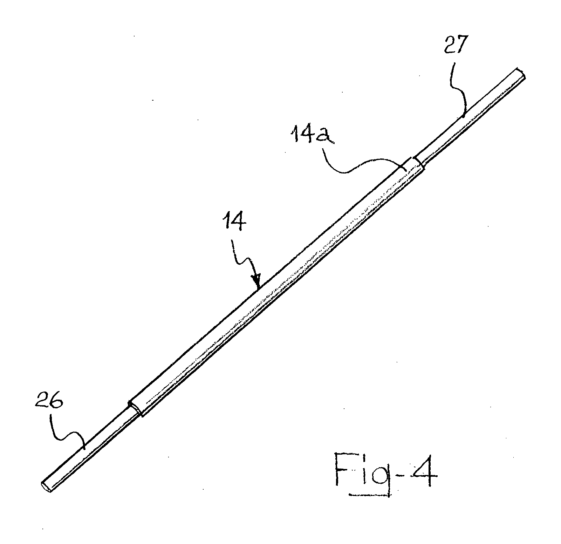

Referring first to figures 1, 2a, 2b, 3a, 3b and 4, an intramedullary nail according to the present invention is shown and globally indicated with 10, suitable for insertion in a fractured elongate bone 12, such as for example a femur or a tibia.

The nail 10 comprises a substantially straight stem 14 extending between a proximal end 16 and a distal end 18.

The stem 14 is preferably composed of a cylindrical tube. Expansion means 20 for a fixation of the nail 10 to said fractured bone 12 are provided in correspondence with at least one of the two ends 16 and 18.

More precisely, according to an aspect of the present invention, these expansion means 20 comprise at least an element 20a realised with a shape-memory material.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory materials suitable for use in the present invention are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

In the example of figure 1, expansion means 20 are provided in correspondence with each of the two ends 16 and 18.

An element 20a realised with a shape-memory material of the expansion means 20 of the proximal end 16 is a first cylindrical sleeve 22, preferably equipped with a plurality of longitudinal slots 24 (in the shown example they are four). They are angularly equally spaced around the cylinder of the first sleeve 22, the slots 24 defining a plurality of fillets 24a. The slots 24, at the two opposite longitudinal ends, are shaped according to a circumference arc.

The cylindrical tube of the stem 14 has, in correspondence with the proximal end 16, a first narrow section 26, around which the first sleeve 22 is worn.

The first narrow section 26 has a thread (not shown in the figures) at the stem free end, a retaining ring 28 of the cylindrical sleeve 22 being screwed on said thread, defining a position of the first sleeve 22 with respect to said first narrow section 26.

Moreover, the first sleeve 22 comprises at least one tongue-shaped appendix 30 extending, in a longitudinal direction, towards a stem central portion 14a: the appendix 30 is housed in a corresponding tongue housing 32 provided on the stem and starting from the end of the first narrow section 26 opposite to said free end. Therefore a relative-rotation-free coupling is ensured between the first sleeve 22 and the stem 14.

An element 20a realised with a shape-memory material of the of the expansion means 20 of the distal end 18 is a second cylindrical sleeve 23, preferably equipped with a plurality of longitudinal slots 25 (in the shown example they are four). They are angularly equally spaced around the cylinder of the second sleeve 23, the slots 25 defining a plurality of fillets 25a. The slots 25, at the two opposite longitudinal ends, are shaped according to a circumference arc.

The cylindrical tube of the stem 14 has, in correspondence with the distal end 18, a second narrow section 27, around which the second sleeve 23 is worn.

The second narrow section 27 has a thread (not shown in the figures) at the stem free end, a retaining plug 29 of the cylindrical sleeve 23 being screwed on said thread, defining a position of the second sleeve 23 with respect to said second narrow section 27.

Similarly to the first sleeve 22, the second sleeve 23 comprises at least one tongue-shaped appendix 31 extending, in a longitudinal direction, towards a central portion 14a of the stem: the appendix 31 is housed in a corresponding tongue housing 33 provided on the stem central portion 14a and starting from the end of the second narrow section 27 opposite to said free end. Therefore a relative-rotation-free coupling is ensured between the second sleeve 23 and the stem 14.

The first sleeve 22 and the second sleeve 23 are similar and they are specularly positioned, with the stem tongue housing 32 being angularly positioned in correspondence with the stem tongue housing 33.

The above description corresponds to the initial shape of the expansion means 20 realised with a shape-memory material.

In the final shape, corresponding to the fixation of the nail 10 to said fractured bone 12, said first cylindrical sleeve 22 and said second cylindrical sleeve 23 take a cask configuration outside the narrow sections 26 and 27 respectively, with a subsequent shortening of the longitudinal dimension of the two sleeves 22 and 23; in the preferred embodiment with the fillets 24a and 25a, in the final shape, the fillets 24a and 25a take a cask stave configuration, with a subsequent shortening of the longitudinal dimension of the two sleeves 22 and 23.

The tongue-shaped appendixes 30 and 31, when passing from the initial shape to the final shape, slide in the tongue housings 32 and 33, and also in said final shape the tongue-shape appendixes 30 and 31 are inserted in the corresponding tongue housings 32 and 33.

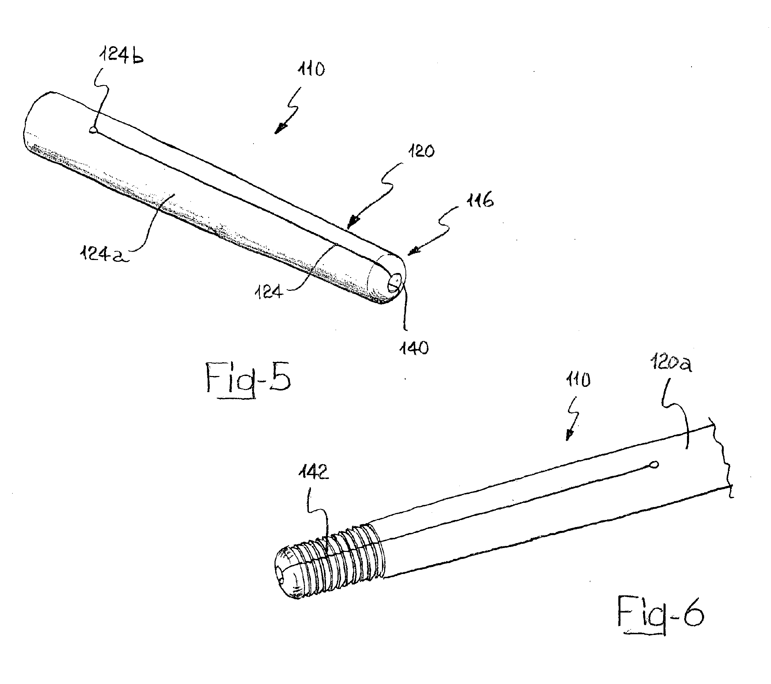

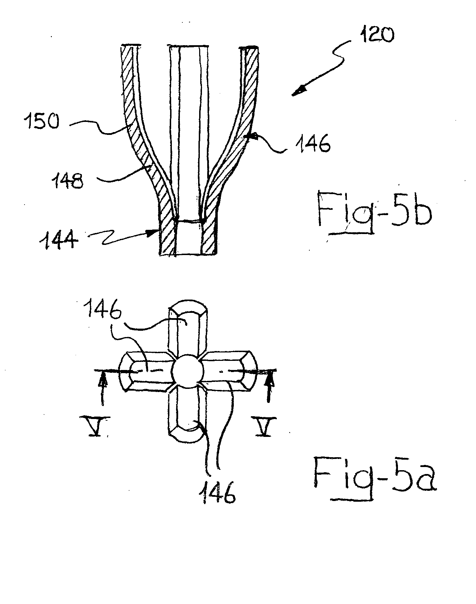

Referring now to figure 5, a second embodiment of intramedullary nail, according to the present invention, is shown and globally indicated with 110, suitable for insertion in a fractured elongate bone 112. In this embodiment, the elements being structurally or functionally similar to the elements of the intramedullary nail 10 are indicated with the same reference number increased by 100.

The nail 110 comprises a substantially straight stem 114 extending between a proximal end 116 and a distal end.

The substantially straight stem 114 preferably comprises a cylindrical sleeve, while expansion means 120 are provided in correspondence with at least one of the two ends for a fixation of the nail 110 to said fractured bone 112.

More precisely, according to an aspect of the present invention, these expansion means 120 comprise at least an element 120a realised with a shape-memory material.

In particular, expansion means 120 of the same type are provided both at the proximal end 116 and at the distal end.

The elements 120a realised with a shape-memory material of the expansion means 120 are, in this case, each of the proximal 116 and distal ends, whereon a plurality of longitudinal cuts 124 are provided, said cuts 124 cutting the thickness of the cylindrical sleeve of the stem 114. The cuts 124 are preferably equally spaced around the stem cylindrical sleeve and they define a plurality of foils 124a. The cuts 124 abut in grooves 124b being shaped according to a circumference arc.

It is specified that the stem cylindrical sleeve is bounded, at the proximal 116 and distal ends, by a bevelled surface 140, split by said cuts 124, said bevelled surface 140 being for example a portion of a spherical cap.

Referring now to figures 5a and 5b, an alternative embodiment of said intramedullary nail 110 is shown.

The elements 120a realised with a shape-memory material of the expansion means 120 are, in this case, two inserts 144 positioned the one at the proximal end 116 and the other at the distal end of the nail 110, in correspondence with respective lowered-cross-section portions of the stem 114.

Each insert 144 is shaped as a cylindrical sleeve comprising, at the end outside the stem 114, a plurality of longitudinal cuts 124, said cuts 124 cutting the thickness of the cylindrical sleeve of the insert 144. The cuts 124 are preferably equally spaced around the cylindrical sleeve of the insert 144 and they define a plurality of tongues 146. In the case shown in the figures the tongues 146 are four. In the initial shape, starting from the cylindrical sleeve without cuts 124, each tongue 146 has a first bend 148 outside the nail 110 and a following second bend 150 inwards the nail 110, so that the end section of the tongue 146 is positioned in a substantially axial direction with respect to the stem 114.

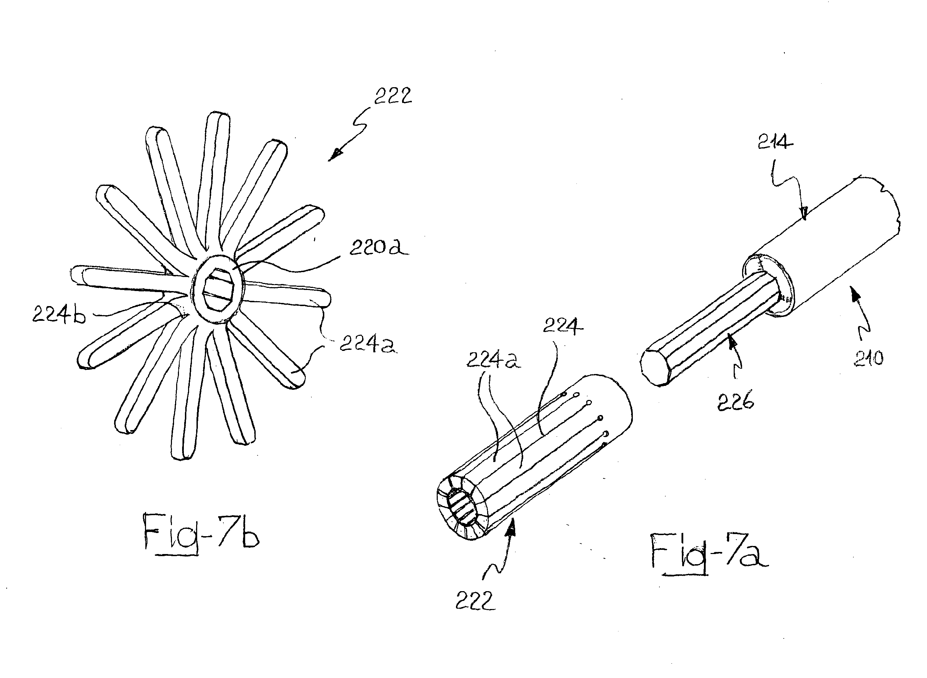

In a further alternative embodiment of said intramedullary nail 110, shown in figure 6, ring-shaped grooves 142 are provided, realised in correspondence with the two ends of the stem cylindrical sleeve: said ring-shaped grooves 142 are broken, along the circumference, by the cut 124 crossing.

The above description corresponds to the initial shape of the expansion means 120 realised with a shape-memory material. In the final shape, the free ends of the foils 124a or of the tongues 146 bend outside the stem, the foils 124a or the tongues 146 thus positioning in a substantially radial way.

Referring now to figures 7a and 7b a third embodiment of intramedullary nail, according to the present invention, is shown and globally indicated with 210, suitable for insertion in a fractured elongate bone 212. In this embodiment, the elements being structurally or functionally similar to the elements of the intramedullary nail 10 are indicated with the same reference number increased by 200.

The nail 210 comprises a substantially straight stem 214 extending between a proximal end and a distal end.

The substantially straight stem 214 preferably comprises a cylindrical sleeve, while expansion means 220 are provided in correspondence with at least one of the two ends for a fixation of the nail 210 to said fractured bone 212.

More precisely, according to an aspect of the present invention, these expansion means 220 comprise at least an element 220a realised with a shape-memory material.

In particular, expansion means 220 of the same type are provided both at the proximal end and at the distal end.

An element 220a realised with a shape-memory material of the expansion means 220 is a cylindrical sleeve 222 comprising a plurality of longitudinal cuts 224 crossing the thickness of the cylindrical sleeve 222, said cuts 224 starting from an end of the sleeve 222 and abutting in corresponding grooves 224b. The cuts 224 are preferably equally spaced around the cylindrical sleeve 222 and they define a plurality of foils 224a. The grooves 224b of the cuts 224 are shaped according to a circumference arc.

The cylindrical sleeve of the stem 214 has, in correspondence with each end, a narrow section 226, wherein the cylindrical sleeve 222 is worn, so that the free end of the foils 224a is turned outside the stem. More precisely, in the example of figures 7a and 7b, the narrow section 226 has an outer prismatic shape, for example in the shape of a regular octagonal prism, corresponding to an inner conjugated prismatic shape, for example in the shape of a regular octagonal prism, of the central hole of the cylindrical sleeve 222.

It is specified that the cylindrical sleeve 222, so worn in the narrow section 226, is fixed therein, using for the fixation an area of the cylindrical sleeve 222 being opposite to the starting end of said cuts 224.

The above description corresponds to the initial shape of the expansion means 220 realised with a shape-memory material. In the final shape, the free ends of the foils 224 bend outside the stem, the foils 224a thus positioning in a substantially radial way.

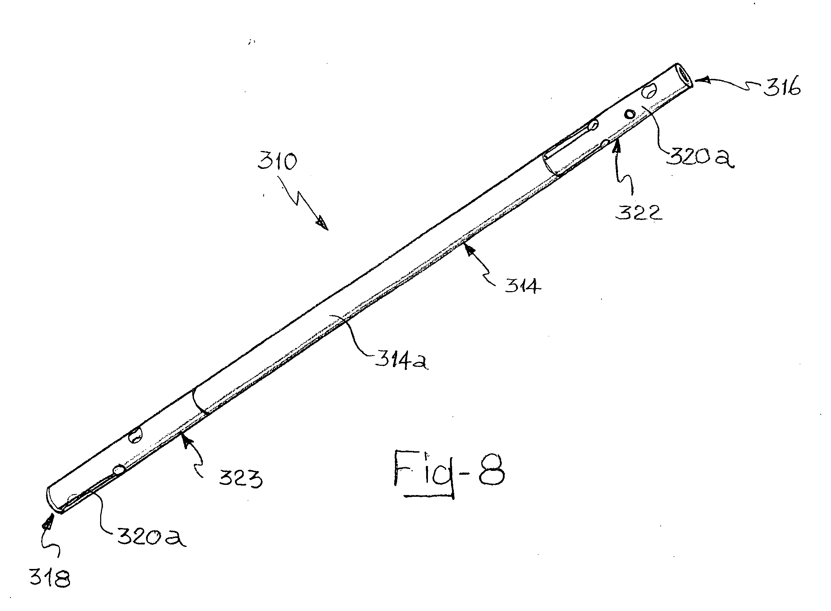

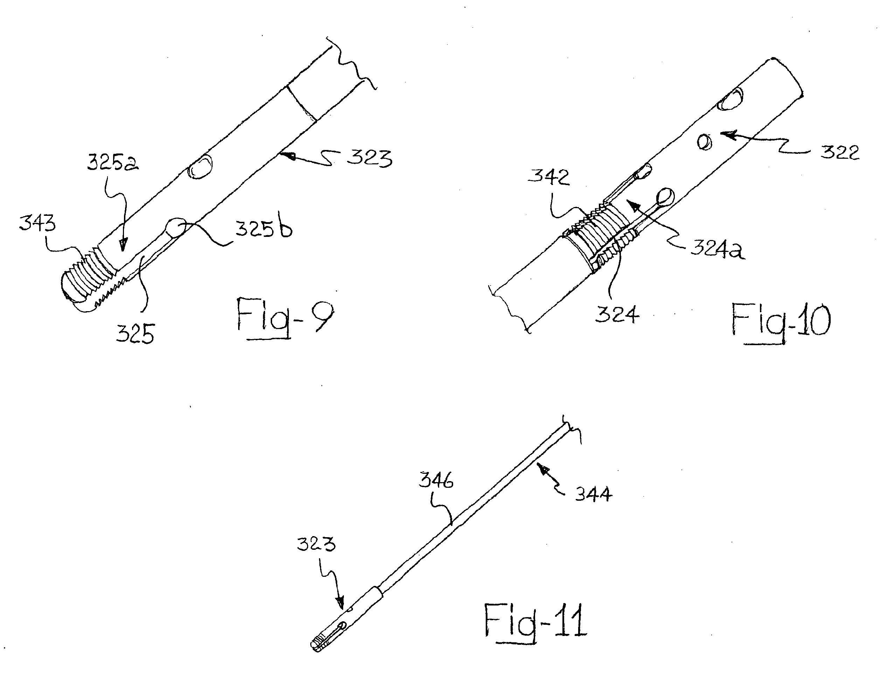

Referring now to figures 8, 9, 10 and 11, a forth embodiment of intramedullary nail, according to the present invention, is shown and globally indicated with 310, suitable for insertion in a fractured elongate bone 312. In this embodiment, the elements being structurally or functionally similar to the elements of the intramedullary nail 10 are indicated with the same reference number increased by 300.

The nail 310 comprises a substantially straight stem 314 extending between a proximal end 316 and a distal end 318.

The substantially straight stem 314 is preferably composed of a cylindrical tube, while expansion means 320 are provided in correspondence with at least one of the two ends 316 and 318 for a fixation of the nail 310 to said fractured bone 312.

In particular, in the example of figure 8, expansion means 320 are provided both at the proximal end 316 and at the distal end 318.

An element 320a realised with a shape-memory material of the expansion means 320, for example of the proximal end 316, is a first cylindrical sleeve 322 comprising a plurality of longitudinal cuts 324 crossing the thickness of the cylindrical sleeve 322, said cuts 324 starting from an end of the sleeve 322 and abutting in corresponding grooves 324b. The cuts 324 are preferably equally spaced around the cylindrical sleeve 322 and they define a plurality of foils 324a. In the example of the figures, the foils 324 are four; the grooves 324b of the cuts 324 are shaped according to a circumference arc. Ring-shaped grooves 342 are also provided, realised in correspondence with the free ends of the foils 324a: said ring-shaped grooves 342 are broken, along the circumference, by the cut 324 crossing.

The cylindrical tube of the stem 314 has, in correspondence with the proximal end 316, a first narrow section 326, wherein the cylindrical sleeve 322 is worn, so that the free end of the foils 324a is turned towards a stem central portion 314a and it abuts in correspondence with the stem outer diameter change, wherein the first narrow section 326 is provided. Preferably, the first narrow section 326 has an outer prismatic shape corresponding to an inner conjugated prismatic shape of the central hole of the first cylindrical sleeve 322.

The foils 324a are realised with a shape-memory material and the first cylindrical sleeve 322, worn in the first narrow section 326, is fixed therein, using for the fixation an area of the cylindrical sleeve 322 being opposite to the starting end of said cuts 324.

The above description corresponds to the initial shape of the foils 324a. In the final shape, the free ends of the foils 324a bend outside the stem, the foils 324a thus positioning in a substantially radial way.

According to an alternative embodiment, the foils 324a are not realised with a shape-memory material. In this case, in the final shape, the first cylindrical sleeve 322 is moved towards a stem central portion 314a by driving means 344, so that the foils 324a are bent outside the first narrow section 326, similarly to the fillets 24a and 25a of the first embodiment of the nail 10.

Driving means 344 are realised for example with a cylinder 346, made of a shape-memory material, which shortens in the final shape, inserted in the stem hole and fixed in said stem and in said first cylindrical sleeve 322.

An element 320a realised with a shape-memory material of the expansion means 320, for example of the distal end 318, is a second cylindrical sleeve 323 comprising a plurality of longitudinal cuts 325 crossing the thickness of the cylindrical sleeve 323, said cuts 325 starting from an end of the sleeve 323 and abutting in corresponding grooves 325b. The cuts 325 are preferably equally spaced around the cylindrical sleeve 323 and they define a plurality of foils 325a. In the example of the figures, the foils 324 are two; the grooves 325b of the cuts 325 are shaped according to a circumference arc. Ring-shaped grooves 343 are also provided, realised in correspondence with the free ends of the foils 325a: said ring-shaped grooves 343 are broken, along the circumference, by the cut 325 crossing.

The cylindrical tube of the stem 314 has, in correspondence with the distal end 318, a second narrow section 327, wherein the cylindrical sleeve 323 is worn, so that the free end of the foils 325a is turned outside the stem. Preferably, the narrow section 327 has an outer prismatic shape corresponding to an inner conjugated prismatic shape of the central hole of the cylindrical sleeve 323.

It is specified that the cylindrical sleeve 323, so worn in the narrow section 327, is fixed therein, using for the fixation an area of the cylindrical sleeve 323 being opposite to the starting end of said cuts 325.

The above description corresponds to the initial shape of the second cylindrical sleeve 323. In the final shape, the free ends of the foils 325a, which, according to an aspect of the present invention, are realised with a shape-memory material, bend outside the stem, the foils 325a thus positioning in a substantially radial way.

It must be noticed that the cylinder 346 of the driving means 344 is, in the example of figure 11, realised enbloc with the second cylindrical sleeve 323, and it is practically an extension thereof. In this case, the cylinder 346 is obviously not directly fixed to the stem, since the fixation indirectly occurs by means of the second cylindrical sleeve 323 which, as already mentioned, is directly fixed to the second narrow section 327 of the stem. Alternatively, the fixation of the second cylindrical sleeve 323 to the second narrow section 327 can be omitted, providing a correct length for the cylinder 346, i.e. such a length as to make the fist cylindrical sleeve 322 and the second cylindrical sleeve 323 abut in correspondence with the two stem outer diameter changes, wherein the first narrow section 326 and the second narrow section 327 respectively are provided.

An application method, according to the present invention, of said intramedullary nail in said elongate bone, comprises:
a positioning step of said nail in said elongate bone, to mend the fracture;
an expansion step, with the nail fixation to said bone, of said at least one of the two nail ends, such end being equipped with expansion means.

More precisely, according to an aspect of the present invention, said expansion step corresponds to an operation step of an element realised with a shape-memory material and comprised in said expansion means.

The operation of the intramedullary nail, suitable for insertion in a fractured elongate bone, according to the present invention, is already partially evident from the description of the above application method and it is specified hereafter.

In the case of the first embodiment of the intramedullary nail 10, the nail 10 is positioned in said elongate bone 12, in the initial shape thereof, with the fillets 24a and 25a of the two cylindrical sleeves 22 and 23 having a straight shape.

Afterwards, in order to fix the nail 10 to said bone 12, said expansion means 20 are operated, so that the fillets 24a and 25a of the first cylindrical sleeve 22 and of the second cylindrical sleeve 23 respectively tend to bend outside the first narrow section 26 and the second narrow section 27, with a subsequent interference of the fillets 24a and 25a with respect to the bone portion surrounding said first cylindrical sleeve 22 and said second sleeve 23 respectively: this interference practically causes a grip, solidly fixing said nail 10 in the bone 12.

The tongue-shaped appendixes 30 and 31, when passing from the initial shape to the final shape, slide in the tongue housings 32 and 33 of the stem, in said final shape the tongue-shape appendixes 30 and 31 being inserted in the corresponding tongue housings 32 and 33, so as to avoid undesired torsions or rotations of the nail 10. Moreover, since the tongue-shaped appendixes 30 and 31 have continuous straight profiles, an easy sliding of the same in the tongue housings 32 and 33 is allowed during the initial instruction step of the shape-memory material.

It must be noticed that the ring 28, besides stopping the movement of the first cylindrical sleeve 22, can be connected to an outer template.

In the case of the second embodiment of the intramedullary nail 110, the nail 110 is positioned in said elongate bone 112, in the initial shape thereof, with the foils 124a of the two stem ends having a straight shape.

Afterwards, in order to fix the nail 110 to said bone 112, said expansion means 120 are operated, so that the foils 124a tend to bend outside the stem, with a subsequent interference of the foils 124a with respect to the bone portion surrounding said proximal and distal ends of said nail 110: this interference practically causes a grip, solidly fixing said nail 110 in the bone 112.

The ring-shaped grooves 142 serve to improve the grip, practically preventing the nail 110 from sliding in the bone 112 during the operation of expansion means 120.

In the case of the third embodiment of the intramedullary nail 210, the nail 210 is positioned in said elongate bone 212, in the initial shape thereof, with the foils 224a of the two cylindrical sleeves 222 having a straight shape.

Afterwards, in order to fix the nail 210 to said bone 212, said expansion means 220 are operated, so that the foils 224a tend to bend outside the cylindrical sleeves 222, with a subsequent interference of the foils 224a with respect to the bone portion surrounding said proximal and distal ends of said nail 210: this interference practically causes a grip, solidly fixing said nail 210 in the bone 212.

It must be noticed that the narrow section 226 has an outer prismatic shape, corresponding to an inner conjugated prismatic shape of the central hole of the cylindrical sleeve 222, to prevent undesired torsions or rotations of the nail 210.

In the case of the forth embodiment of the intramedullary nail 310, the nail 310 is positioned in said elongate bone 312, in the initial shape thereof, with the foils 324a and 325a of the first 322 and second cylindrical sleeve 323 respectively having a straight shape.

Afterwards, in order to fix the nail 310 to said bone 312, said expansion means 320 are operated, so that the foils 324a and 325a tend to bend outside the cylindrical sleeves 322 and 323, with a subsequent interference of the foils 324a and 325a with respect to the bone portion surrounding said proximal 316 and distal 318 ends of said nail 310: this interference practically causes a grip, solidly fixing said nail 310 in the bone 312.

It must be noticed that the stem narrow sections 326 and 327 have an outer prismatic shape, corresponding to an inner conjugated prismatic shape of the central hole of the cylindrical sleeves 322 and 323 respectively, to prevent undesired torsions or rotations of the nail 310.

The ring-shaped grooves 342 and 343 serve to improve the grip, practically preventing the nail 310 from sliding in the bone 312 during the operation of expansion means 320.

In the alternative embodiment of the nail 310 wherein the foils 324a are not realised with a shape-memory material, in the final shape, the first cylindrical sleeve 322 is moved towards a stem central portion 314a by driving means 344, so that the foils 324a tend to bent outside the first narrow section 326, causing the same above-mentioned interference effect with the bone 312.

The main advantage achieved by the intramedullary nail suitable for insertion in a fractured elongate bone, as well as by the application method of said nail in said bone, according to the present invention, is the fact of unusually simplifying the fixation step of the intramedullary nail inserted in the bone: the expansion of expansion means occurs in fact without a mechanical manual intervention, the shape-memory material used in the nail of the invention expanding instead by heat absorption. In practise, the nail fixation is obtained simultaneously with the insertion thereof in the bone, since the shape-memory elements, cooled at first to take the initial shape, take in the bone their final shape, due to the heat released by the patient's body.

Another advantage of the intramedullary nail suitable for insertion in a fractured elongate bone, according to the present invention, is to prevent undesired nail torsions or rotations, since the tongue-shaped appendix is held in the housing thereof.

Another advantage of the intramedullary nail according to the present invention is to preserve the length of the bone on which the intervention occurs, since the tongue-shaped appendix slides in the housing thereof, the nail stem thus keeping the starting position thereof.

A further advantage of the intramedullary nail according to the present invention is that, in case of bone lyses in the contact area between the expanded cylindrical sleeves and the bone, the nail fixation is however ensured, with no need for an external intervention, since the final shape of the cylindrical sleeves realised with a shape-memory material have a higher radial dimension than the radial dimension of the cylindrical sleeves in the fixation step of the nail to the bone, so that a considerable contact pressure is preserved.

A still further advantage of the intramedullary nail according to the present invention is to be easy to produce, because of the reduced number of different pieces (reminding that the two cylindrical sleeves are identical).

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110, 210, 310) suitable for insertion in a fractured elongate bone (12, 112, 212, 312), comprising a substantially straight stem (14, 114, 214, 314) extending between a proximal end (16, 116, 216, 316) and a distal end (18, 118, 218, 318), at least one of said ends (16, 18; 116, 118; 216, 218; 316, 318) comprising expansion means (20, 120, 220, 320) for the nail fixation to the bone, **characterised in that** said expansion elements (20, 120, 220, 320) comprise at least an element (20a, 120a, 220a, 320a) realised with a shape-memory material.

2. Intramedullary nail (10) according to claim 1, **characterised in that** said at least one element (20a) is a cylindrical sleeve (22, 23), worn in correspondence with a narrow section (26, 27) of said at least one end (16, 18), said cylindrical sleeve (22, 23) taking a cask configuration for the nail fixation to the bone.

3. Intramedullary nail (10) according to claim 2, **characterised in that** said cylindrical sleeve (22, 23) comprises a plurality of longitudinal slots (24, 25), the slots (24, 25) defining a plurality of fillets (24a, 25a), said fillets (24a, 25a) taking a cask stave configuration for the nail fixation to the bone.

4. Intramedullary nail (10) according to claim 3, **characterised in that** said slots (24, 25) are angularly equally spaced.

5. Intramedullary nail (10) according to claim 3, **characterised in that** the two opposite longitudinal ends of the slots (24, 25) are shaped.

6. Intramedullary nail (10) according to claim 3 **characterised in that** said slots (24, 25) are shaped according to a circumference arc.

7. Intramedullary nail (10) according to claim 2, **characterised in that** said narrow section (26) has a thread at the stem free, a retaining ring (28) of the cylindrical sleeve (22) being screwed on said thread.

8. Intramedullary nail (10) according to claim 2, **characterised in that** said narrow section (27) has a thread at the stem free, a retaining plug (29) of the cylindrical sleeve (23) being screwed on said thread.

9. Intramedullary nail (10) according to claim 2, **characterised in that** said sleeve (22, 23) comprises at least one tongue-shaped appendix (30, 31) extending, in a longitudinal direction, towards a stem central portion (14a), the appendix (30, 31) being housed in a corresponding tongue housing (32, 33) provided on the stem central portion (14a) and starting from the end of the narrow section (26, 27) opposite to the stem free end.

10. Intramedullary nail (10) according to claim 2, **characterised in that** a cylindrical sleeve (22, 23) is provided at each end (16, 18) of the stem (14), said sleeves (22, 23) being identical and specularly positioned.

11. Intramedullary nail (10) according to claim 1, **characterised in that** said substantially straight stem (14) is a cylindrical tube.

12. Intramedullary nail (110) according to claim 1, **characterised in that** said substantially straight stem (114) is a cylindrical tube and **in that** said elements (120a) realised with a shape-memory material of the expansion means (120) are each of said proximal (116) and distal ends, whereon a plurality of longitudinal cuts (124) are provided, said cuts (124) cutting the thickness of the cylindrical sleeve of the stem (114), defining a plurality of foils (124a). and abutting in grooves (124b), the free ends of the foils (124a) bending outside the stem (114) in a final shape corresponding to the fixation of the nail (110) to said fractured bone (112).

13. Intramedullary nail (110) according to claim 1, **characterised in that** said substantially straight stem (114) is a cylindrical tube and **in that** said elements (120a) realised with a shape-memory material of the expansion means (120) are two inserts (144) positioned the one at the proximal end (116) and the other at the distal end of the nail (110), in correspondence with respective lowered-cross-section portions of the stem (114), each insert (144) being shaped as a cylindrical sleeve comprising, at the end outside the stem (114), a plurality of longitudinal cuts (124), said cuts (124) cutting the thickness of the cylindrical sleeve of the insert (144) and defining a plurality of tongues (146), each tongue (146), starting from the cylindrical sleeve without cuts (124), having a first bend (148) outside the nail (110) and a following second bend (150) inwards the nail (110), so that the end section of the tongue (146) is positioned in a substantially axial direction with respect to the stem (114), the free ends of the tongues (146) bending outside the stem (114) in a final shape corresponding to the fixation of the nail (110) to said fractured bone (112).

14. Intramedullary nail (110) according to claims 12 or 13, **characterised in that** said cuts (124) are equally spaced.

15. Intramedullary nail (110) according to claim 12, **characterised in that** said grooves (124b) of said cuts (124) are shaped.

16. Intramedullary nail (110) according to claim 12, **characterised in that** said grooves (124b) of said cuts (124) are shaped according to a circumference arc.

17. Intramedullary nail (110) according to claim 12, **characterised in that** said stem cylindrical sleeve is bounded, at the proximal (116) and distal ends, by a bevelled surface (140), split by said cuts (124).

18. Intramedullary nail (110) according to claim 16, **characterised in that** said bevelled surface (140) is a portion of a spherical cap.

19. Intramedullary nail (110) according to claim 12, **characterised in that** ring-shaped grooves(142) are provided, realised in correspondence with the two ends of the stem cylindrical tube.

20. Intramedullary nail (210) according to claim 1, **characterised in that** said at least one element (220a) of said expansion means (220) is a cylindrical sleeve (222) comprising a plurality of longitudinal cuts (224) crossing the thickness of the cylindrical sleeve (222), said cuts (224) starting from an end of the sleeve (222) and abutting in corresponding grooves (224b), said cuts (224) defining a plurality of foils (224a), said stem (214) having, in correspondence with said at least one end (216, 218), a narrow section (226), wherein said cylindrical sleeve (222) is worn, so that the free end of the foils (224a) is turned outside the stem, the free ends of the foils (224a) bending outside the stem (214) for the nail fixation to the bone.

21. Intramedullary nail (210) according to claim 20, **characterised in that** said cuts (224) are equally spaced.

22. Intramedullary nail (210) according to claim 20, **characterised in that** said grooves (224b) of said cuts (224) are shaped.

23. Intramedullary nail (210) according to claim 20, **characterised in that** said grooves (224b) of said cuts (224) are shaped according to a circumference arc.

24. Intramedullary nail (210) according to claim 20, **characterised in that** said narrow section (226) has an outer prismatic shape corresponding to an inner conjugated prismatic shape of the central hole of the cylindrical sleeve (222).

25. Intramedullary nail (210) according to claim 24, **characterised in that** said outer prismatic shape of said narrow section (226) and of said central hole of the cylindrical sleeve (222) is a regular octagonal prism.

26. Intramedullary nail (210) according to claim 20, **characterised in that** the cylindrical sleeve (222), worn in the narrow section (226), is fixed therein.

27. Intramedullary nail (210) according to claim 26, **characterised in that**, for the fixation of said cylindrical sleeve (222) in the narrow section (226), an area of the cylindrical sleeve (222) being opposite to the starting end of said cuts (224) is used.

28. Intramedullary nail (310) according to claim 1, **characterised in that** said at least one element (320a) is a cylindrical sleeve (322) comprising a plurality of longitudinal cuts (324) crossing the thickness of the cylindrical sleeve (322), said cuts (324) starting from an end of the sleeve (322) and abutting in corresponding grooves (324b), said cuts (324) defining a plurality of foils (324a), said stem (314) having, in correspondence with said at least one end (316), a narrow section (326), wherein said cylindrical sleeve (322) is worn, so that the free end of the foils (324a) is turned towards a stem central portion (314a), the free ends of the foils (324a) bending outside the stem (314) in a final shape corresponding to the fixation of the nail (310) to said fractured bone (312).

29. Intramedullary nail (310) according to claim 1, **characterised in that** said at least one element (320a) of said expansion means (320) is a cylindrical sleeve (323) comprising a plurality of longitudinal cuts (325) crossing the thickness of the cylindrical sleeve (323), said cuts (325) starting from an end of the sleeve (323) and abutting in corresponding grooves (325b), said cuts (325) defining a plurality of foils (325a), said stem (314) having, in correspondence with said at least one end (318), a narrow section (327), wherein said cylindrical sleeve (323) is worn, so that the free end of the foils (325a) is turned outside the stem, the free ends of the foils (325a) bending outside the stem (314) in a final shape corresponding to the fixation of the nail (310) to said fractured bone (312).

30. Intramedullary nail (310) according to claims 28 or 29, **characterised in that** said cuts (324, 325) are equally spaced.

31. Intramedullary nail (310) according to claims 28 or 29, **characterised in that** said grooves (324b, 325b) of said cuts (324, 325) are shaped.

32. Intramedullary nail (310) according to claims 28 or 29, **characterised in that** said grooves (324b, 325b) of said cuts (324, 325) are shaped according to a circumference arc.

33. Intramedullary nail (310) according to claims 28 or 29, **characterised in that** ring-shaped grooves (342, 343) are provided, realised in correspondence with the free ends of the foils (324a, 325a).

34. Intramedullary nail (310) according to claims 28 or 29, **characterised in that** said narrow section (326, 327) has an outer prismatic shape corresponding to an inner conjugated prismatic shape of the central hole of the cylindrical sleeve (322, 323).

35. Intramedullary nail (310) according to claims 28 or 29, **characterised in that** the cylindrical sleeve (322, 323), worn in the narrow section (326, 327), is fixed therein.

36. Intramedullary nail (310) according to claims 28 or 29, **characterised in that**, for the fixation of said cylindrical sleeve (322, 323) in the narrow section (326, 327), an area of the cylindrical sleeve (322, 323) being opposite to the starting end of said cuts (324, 325) is used.

37. Intramedullary nail (310) according to claim 28, **characterised in that** said cylindrical sleeve (322) abuts in correspondence with the stem outer diameter change, wherein the narrow section (326) is provided.

38. Intramedullary nail (310) according to claim 29, **characterised in that**, in correspondence with the other stem end (316), opposite to the end wherein said cylindrical sleeve (323) is provided, a further cylindrical sleeve (322) is provided, comprising a plurality of longitudinal cuts (324) crossing the thickness of the further cylindrical sleeve (322), said cuts (324) starting from an end of the sleeve (322) and abutting in corresponding grooves (324b), said cuts (324) defining a plurality of foils (324a), said stem (314) having, in correspondence with said otehr end (316), a narrow section (326), wherein said further cylindrical sleeve (322) is worn, so that the free end of the foils (324a) is turned towards a stem central portion (314a), in a final shape corresponding to the fixation of the nail (310) to said fractured bone (312), said further cylindrical sleeve (322) being moved towards a stem central portion (314a) by driving means (344), so that the free ends of the foils (324a) bend outside the stem (314).

39. Intramedullary nail (310) according to claim 38, **characterised in that** the substantially straight stem (314) is a cylindrical sleeve and **in that** said driving means (344) are realised with a cylinder (346), made of shape-memory material, which shortens in the final shape, said cylinder (346) being inserted in the stem hole and fixed in said stem and in said further cylindrical sleeve (322).

40. Intramedullary nail (310) according to claim 38, **characterised in that** the substantially straight stem (314) is a cylindrical sleeve and **in that** said driving means (344) are realised with a cylinder (346), made of a shape-memory material, which shortens in the final shape, said cylinder (346) being inserted in the stem hole and being fixed in said further cylindrical sleeve (322), said cylinder (346) being realised enbloc with said cylindrical sleeve (323) and being an extension thereof, the cylindrical sleeve (323), worn in the narrow section (326, 327), being fixed thereto.

41. Intramedullary nail (310) according to claim 38, **characterised in that** the substantially straight stem (314) is a cylindrical sleeve and **in that** said driving means (344) are realised with a cylinder (346), made of a shape-memory material, which shortens in the final shape, said cylinder (346) being inserted in the stem hole and being fixed in said further cylindrical sleeve (322), said cylinder (346) being realised enbloc with said cylindrical sleeve (323) and being an extension thereof, such a length being provided, for the cylinder (346), as to make said further cylindrical sleeve (322) and said cylindrical sleeve (323) abut in correspondence with the two stem outer diameter changes, wherein said two narrow sections (326, 327) are respectively provided.

42. Intramedullary nail (10, 110, 210, 310) according to claim 1, **characterised in that** said elongate bone (12, 112, 212, 312) is a femur.

43. Intramedullary nail (10, 110, 210, 310) according to claim 1, **characterised in that** said elongate bone (12, 112, 212, 312) is a tibia.

44. Application method, in a fractured elongate bone (12, 112, 212, 312), of an intramedullary nail (10, 110, 210, 310) according to claim 1, **characterised in that** it comprises:
a positioning step of said nail (10, 110, 210, 310) in said elongate bone (12, 112, 212, 312), to mend the fracture;
an expansion step, with the fixation of the nail (10, 110, 210, 310) to said bone (12, 112, 212, 312), of said end (16, 18; 116, 118; 216, 218; 316, 318) of the nail (10, 110, 210, 310) equipped with said expansion means (20, 120, 220, 320).

45. An application method according to claim 44, **characterised in that** said expansion step corresponds to an operation step of an element (20a, 120a, 220a, 320a) realised with a shape-memory material comprised in said expansion means (20, 120, 220, 320).
